# EUROPEAN PATENT APPLICATION

(11) **EP 3 320 948 A1**
(43) Date of publication of application: **16.05.2018**
(21) Application number: 17200932.6
(22) Date of filing: 09.11.2017
(51) Int. Cl.: A61N 1/32, A61N 7/00

(54) **COMPOSITE DEVICE OF ULTRASOUND AND MICROCURRENT FOR SKIN CARE**

(30) Priority: 12.11.2016 JP 2016221070
(71) Applicant: Create Co., Ltd, Fukuoka-shi, Fukuoka 815-0075 (JP); Rited Company Ltd., Gyeonggi-do (KR)
(72) Inventor: PARK, Rae Eun, Seongnam-si, Gyeonggi-do (KR)
(74) Representative: Hewett, Jonathan Michael Richard

(57) **Abstract**

Problem: To provide, as a replacement of a high-intensity focused ultrasound device that is a large and expensive medical instrument/device, a beauty instrument compositing an ultrasound function and a microcunent function that is low-price, small, portable, and suited for everyday use.

Resolution: Provided are a main body (100), a first display unit (200) equipped to a main-body front surface, a button unit (300) equipped to a main-body side surface, a control-circuit unit (400) disposed inside the main body, a cartridge unit (500) mounted inserted into a main-body upper portion, and a head unit (600) that is equipped to a lower portion of the main body and is for irradiating an ultrasound or a microcurrent.

## Description

### FIELD

The present invention relates to a device whose object is skin care and relates more particularly to a composite device compositing a function of irradiating an ultrasound and a function of supplying a microcurrent.

### BACKGROUND

In recent years, interest in skin care is on the rise, particularly with regard to maintaining elasticity-for example, improving facial-skin sagging and wrinkles.

As a medical surgical instrument for removing a sagging area or wrinkles via regenerating or improving facial-skin elasticity, there is an invasive bipolar high-frequency wave, and as a medical surgery, there is a surgical method such as a facelift using string, but recently, as a noninvasive method with no post-surgery downtime, a surgical instrument utilizing high-intensity focused ultrasound (HIFU) technology is also used often.

This high-intensity focused ultrasound technology sets a focus at a certain depth from a surface of skin tissue and induces an action of focusing energy to the focus. By this, energy is transmitted into the dermal layer by setting an appropriate intensity so no thermal denaturation such as thermocoagulation arises to damage the epidermal layer of a surgical subject. A micro thermocoagulation area is formed via the transmitted energy, and elasticity is improved by the skin regenerating via a progressive collagen-generating action.

Such high-intensity focused ultrasound technology drives an ultrasound vibrator based on a piezoelectric element; however, because a piezoelectric vibrator must be actuated under conditions of a high voltage and a high current, a problem is had where operation thereof requires many devices, increasing a device size.

This device is provided with a beauty instrument combining a device that generates a high voltage and a high current and various types of control instruments, the beauty instrument actuating by being connected to a cartridge mounted with a ceramic vibrator. For ultrasound surgery, it is essential to use a cartridge linked to a device whose position is fixed; thus, there is also a problem where a special operator is required to operate this device.

As such a device, the art in the following is known.
JP 2013-146598 A
JP 2014-14516 A

Under these circumstances, a market need is rising for a low-energy and reduced-size instrument that is driven by a principle similar to the above, is not expensive, and can be used as a beauty instrument for everyday use.

### SUMMARY

The present invention is made to meet such a need, and an object thereof is to provide a composite device that is a beauty instrument compositing an ultrasound (moderate-intensity focused ultrasound: MIFU) function and a microcurrent function that is low-price, small, portable, and suited for everyday use to replace a high-intensity focused ultrasound device that is an expensive and large medical instrument/device.

As a means of achieving the object above, a composite device of an ultrasound and a microcurrent for skin care according to claim 1 is provided with a main body, a first display unit equipped to a main-body front surface, a button unit equipped to a main-body side surface, a control-circuit unit disposed inside the main body, a cartridge unit mounted inserted into a main-body upper portion, and a head unit that is equipped to the main body and is for irradiating an ultrasound or a microcurrent to the skin.

In a composite device of an ultrasound and a microcurrent for skin care according to claim 2, in the composite device of an ultrasound and a microcurrent for skin care according to claim 1, the head unit is provided with a conductor unit for flowing the microcurrent by contacting the skin and a sensor unit that senses contact with the skin.

In a composite device of an ultrasound and a microcurrent for skin care according to claim 3, in the composite device of an ultrasound and a microcurrent for skin care according to claim 2, the sensor unit consists of an optical sensor or a contact sensor and a configuration is such that the ultrasound is irradiated only in a situation where contact with the skin is sensed by the sensor unit.

In a composite device of an ultrasound and a microcurrent for skin care according to claim 4, in the composite device of an ultrasound and a microcurrent for skin care according to claim 1, the cartridge unit is configured from an external cartridge filled with an ultrasound medium and an internal cartridge that is disposed inside the external cartridge, receives an ultrasound drive signal, and generates the ultrasound.

In a composite device of an ultrasound and a microcurrent for skin care according to claim 5, in the composite device of an ultrasound and a microcurrent for skin care according to claim 4, the external cartridge has a linking portion that receives from the main body supply of an electrical signal necessary for ultrasound vibration, a medium reservoir filled with the ultrasound medium, and a cartridge case that has the linking portion provided thereto and is mounted to the medium reservoir so the ultrasound medium does not leak to the outside, the composite device further provided with a transmission film that transmits to the skin the ultrasound, which is transmitted through the ultrasound medium, and a ring that prevents the ultrasound medium from leaking to the outside and fixes the transmission film.

In a composite device of an ultrasound and a microcurrent for skin care according to claim 6, in the composite device of an ultrasound and a microcurrent for skin care according to claim 5, the medium reservoir is of a shape where an interior narrows the farther it is from the cartridge case.

In a composite device of an ultrasound and a microcurrent for skin care according to claim 7, in the composite device of an ultrasound and a microcurrent for skin care according to claim 5, the internal cartridge is provided with an ultrasound-generating vibration element of a dome shape where an upper-portion side is expanded in an arc.

In a composite device of an ultrasound and a microcurrent for skin care according to claim 8, in the composite device of an ultrasound and a microcurrent for skin care according to claim 7, the ultrasound-generating vibration element is provided with a size where a diameter is 2 mm or more and 20 mm or less.

In a composite device of an ultrasound and a microcurrent for skin care according to claim 9, in the composite device of an ultrasound and a microcurrent for skin care according to claim 4, any medium among a silicone gel, a silicone oil, and deionized water is filled between an ultrasound-generating vibration element of the external cartridge and a film portion.

In a composite device of an ultrasound and a microcurrent for skin care according to claim 10, in the composite device of an ultrasound and a microcurrent for skin care according to claim 1, the first display unit is provided with an irradiation-count display unit that displays a number of times the ultrasound is irradiated and a state-display unit that displays a supply state of the ultrasound or the microcurrent.

In a composite device of an ultrasound and a microcurrent for skin care according to claim 11, the composite device of an ultrasound and a microcurrent for skin care according to claim 5 is further provided with a second display unit in a cartridge-case upper portion that displays a number of remaining ultrasound irradiations of the cartridge and provides information on a cartridge replacement time.

In a composite device of an ultrasound and a microcurrent for skin care according to claim 12, in the composite device of an ultrasound and a microcurrent for skin care according to claim 1, the cartridge unit is detachable from the main body.

In a composite device of an ultrasound and a microcurrent for skin care according to claim 13, in the composite device of an ultrasound and a microcurrent for skin care according to claim 1, disposed inside the main body is an acceleration sensor that senses movement or a position of the main body and the acceleration sensor senses the movement or the position of the main body when the ultrasound is irradiated, the ultrasound being stopped upon being irradiated once in a situation where it is sensed that the movement of the main body is stopped.

In a composite device of an ultrasound and a microcurrent for skin care according to claim 14, in the composite device of an ultrasound and a microcurrent for skin care according to claim 1, the control unit is provided with a power supply unit that receives power supply from an external power source and supplies an operating voltage to each block configuring a circuit, a control unit that receives the voltage supplied from the power supply unit and controls actuation of the ultrasound or the microcurrent, a microcurrent generation unit that forms a microcurrent generation signal for supplying the microcurrent to the skin, and an ultrasound generation unit that forms an ultrasound generation signal to supply the ultrasound to the skin and an ultrasound amplification unit that receives the signal of the ultrasound generation unit and amplifies the ultrasound to match a predetermined resonant frequency.

In a composite device of an ultrasound and a microcurrent for skin care according to claim 15, in the composite device of an ultrasound and a microcurrent for skin care according to claim 14, the control-circuit unit is provided with a sensor-actuation monitoring unit that senses an actuation state of the ultrasound amplification unit and transmits a control signal according to the actuation state.

In a composite device of an ultrasound and a microcurrent for skin care according to claim 16, in the composite device of an ultrasound and a microcurrent for skin care according to claim 14, the control-circuit unit is provided with an auto-tracing function for matching, in a situation where a deviation in the resonant frequency arises in the ultrasound amplification unit, the arisen deviation.

In a composite device of an ultrasound and a microcurrent for skin care according to claim 17, in the composite device of an ultrasound and a microcurrent for skin care according to claim 14, the power supply unit has a battery unit that saves the power supplied from the external power source and a function of receiving supply of a voltage from the battery unit and raising or lowering the supplied voltage.

In a composite device of an ultrasound and a microcurrent for skin care according to claim 18, in the composite device of an ultrasound and a microcurrent for skin care according to claim 14, the control unit has a function of stopping actuation of the microcurrent generation unit if the ultrasound generation unit actuates and stopping actuation of the ultrasound generation unit if the microcurrent generation unit actuates.

In a composite device of an ultrasound and a microcurrent for skin care according to claim 19, in the composite device of an ultrasound and a microcurrent for skin care according to claim 14, the control unit has a function of counting an actuation count of the ultrasound amplification unit that actuates according to the ultrasound generation signal.

According to the present invention, ultrasound energy is transmitted to the skin and massaging is performed via the microcurrent on the skin where a sensation of heat remains due to thermal stimulation or thermocoagulation. Therefore, the skin can be regenerated by collagen activation and healthy skin can be maintained.

Furthermore, the composite device is portable, being readily able to be carried, and is not restricted in terms of usage location.

In the composite device of an ultrasound and a microcurrent for skin care according to claim 1 of the present invention, provided is a function of supplying the ultrasound or the microcurrent to the skin; therefore, a beautifying effect of the skin is obtained by actions of the ultrasound and the microcurrent.

Because the configuration is such that the ultrasound is transmitted via the ultrasound medium, the stimulation transmitted to the skin is optimally set by the ultrasound medium.

In the composite device of an ultrasound and a microcurrent for skin care according to claim 2, the head unit is provided with the conductor unit; therefore, the microcurrent is supplied to the skin via this conductor unit. Furthermore, because the head unit is provided with the sensor unit, the device operates only in the situation where contact with the skin is sensed.

In the composite device of an ultrasound and a microcurrent for skin care according to claim 3, the sensor unit is configured by the optical sensor or the contact sensor; therefore, the ultrasound or the microcurrent is supplied only in the situation where contact with the skin with sensed by an action of these sensors.

The composite device of an ultrasound and a microcurrent for skin care according to claim 4 is configured by the external cartridge filled with the ultrasound medium and the internal cartridge that is disposed inside the external cartridge, receives the ultrasound drive signal, and generates the ultrasound; therefore, integrally replacing the ultrasound-generating vibration element is possible by replacing these cartridges.

In the composite device of an ultrasound and a microcurrent for skin care according to claim 5, the linking portion, the medium reservoir, and the transmission film are mounted to the cartridge case; therefore, these can be integrally replaced.

In the composite device of an ultrasound and a microcurrent for skin care according to claim 6, the medium reservoir is of the shape where the interior narrows the farther it is from the cartridge case; therefore, the ultrasound is focused to a tip portion and irradiated efficiently.

In the composite device of an ultrasound and a microcurrent for skin care according to claim 7, the internal cartridge is provided with the ultrasound-generating vibration element having the dome form so as to sink to the upper-portion side; therefore, vibration of the ultrasound is transmitted efficiently.

In the composite device of an ultrasound and a microcurrent for skin care according to claim 8, the ultrasound-generating vibration element is provided with the size where the diameter is 2 mm or more and 20 mm or less; therefore, the device main body is formed to a size suited for portability.

In the composite device of an ultrasound and a microcurrent for skin care according to claim 9, any medium among the silicone gel, the silicone oil, and deionized water is filled between the ultrasound-generating vibration element of the external cartridge and the film portion; therefore, the stimulation transmitted to the skin is optimally adjusted via these media.

In the composite device of an ultrasound and a microcurrent for skin care according to claim 10, the first display unit is provided with the irradiation count display unit that displays the number of times the ultrasound is irradiated and the state display unit that displays the supply state of the ultrasound or the microcurrent; therefore, a usage state of the ultrasound-generating vibration element, a replacement time of the cartridge, and the like can be confirmed.

In the composite device of an ultrasound and a microcurrent for skin care according to claim 11, provided in the cartridge-case upper portion is the second display unit that displays the number of remaining ultrasound irradiations of the cartridge and provides information on the cartridge replacement time; therefore, the usage state of the ultrasound-generating vibration element and the replacement time of the cartridge can be confirmed individually for each cartridge.

In the composite device of an ultrasound and a microcurrent for skin care according to claim 12, the cartridge unit is detachable from the main body above; therefore, the cartridge can be integrally replaced in conjunction with degradation of the ultrasound-generating vibration element and the ultrasound medium.

In the composite device of an ultrasound and a microcurrent for skin care according to claim 13, disposed is the acceleration sensor and the configuration is such that the acceleration sensor senses the movement or the position of the main body when the ultrasound is irradiated, the ultrasound being stopped upon being irradiated once if it is sensed that movement of the main body is stopped; therefore, there is no fear of the ultrasound being irradiated excessively to a specific area.

In the composite device of an ultrasound and a microcurrent for skin care according to claim 14, the control-circuit unit is provided with the power supply unit that receives power supply from the external power source and supplies the operating voltage to each block configuring the circuit, the control unit that receives the voltage supplied from the power supply unit and controls actuation of the ultrasound or the microcurrent, the microcurrent generation unit that forms the microcurrent generation signal for supplying the microcurrent to the skin, and the ultrasound generating unit that forms the ultrasound generation signal to supply the ultrasound to the skin and the ultrasound amplification unit that receives the signal of the ultrasound generation unit and amplifies the ultrasound to match the predetermined resonant frequency; therefore, by these, a portable beauty instrument is realized.

In the composite device of an ultrasound and a microcurrent for skin care according to claim 15, the sensor-actuation monitoring unit is provided; therefore, actuation conditions for generating the control signal of the control unit are sensed by taking into consideration safety of product actuation when actuating the ultrasound amplification unit. That is, the sensor-actuation monitoring unit restricts excessive actuation of the ultrasound by monitoring, for example, a situation where a user induces abnormal usage conditions or excessive actuation on the skin.

In the composite device of an ultrasound and a microcurrent for skin care according to claim 16, the auto-tracing function is provided; therefore, resonant frequencies can be matched by auto-tracing an optimal resonant frequency even if a deviation arises between a resonant frequency amplified by the ultrasound amplification unit and a resonant frequency of the ultrasound-generating vibration element.

In the composite device of an ultrasound and a microcurrent for skin care according to claim 17, the power supply unit is provided with a battery unit that saves the power supplied from the external power source and the function of receiving supply of the voltage from the battery unit and raising or lowering the supplied voltage; therefore, a portable and stable power supply system is constructed.

In the composite device of an ultrasound and a microcurrent for skin care according to claim 18, the control unit is provided with a function of stopping actuation of the microcurrent generation unit if the ultrasound generation unit actuates and stopping actuation of the ultrasound generation unit if the microcurrent generation unit actuates; therefore, either the ultrasound or the microcurrent is supplied to the skin.

In the composite device of an ultrasound and a microcurrent for skin care according to claim 19, the control unit has the function of counting the actuation count of the ultrasound amplification unit that actuates according to the ultrasound generation signal; therefore, a usage state of the ultrasound-generating vibration element and the ultrasound medium in conjunction with operation of the device can be confirmed.

### BRIEF DESCRIPTION OF DRAWINGS

FIG. 1 is a front view of a composite device according to an example of the present invention.
FIG. 2 is a back view of the composite device according to the example of the present invention.
FIG. 3 is a left-side view of the composite device according to the example of the present invention.
FIG. 4 is a block diagram of a control-circuit unit of the composite device according to the example of the present invention.
FIG. 5 is a schematic cross-sectional view of a cartridge unit according to the example of the present invention.
FIG. 6 is a plan view of the cartridge unit according to the example of the present invention.
FIG. 7 is a schematic cross-sectional view of the cartridge unit according to another example.

### DESCRIPTION OF EMBODIMENTS

A desirable example of the present invention is described in detail below with reference to the included drawings.

Note that the specific content of the present invention is not limited to the present example, and any design changes or the like within a scope that does not change the gist of the invention are also included in the present invention.

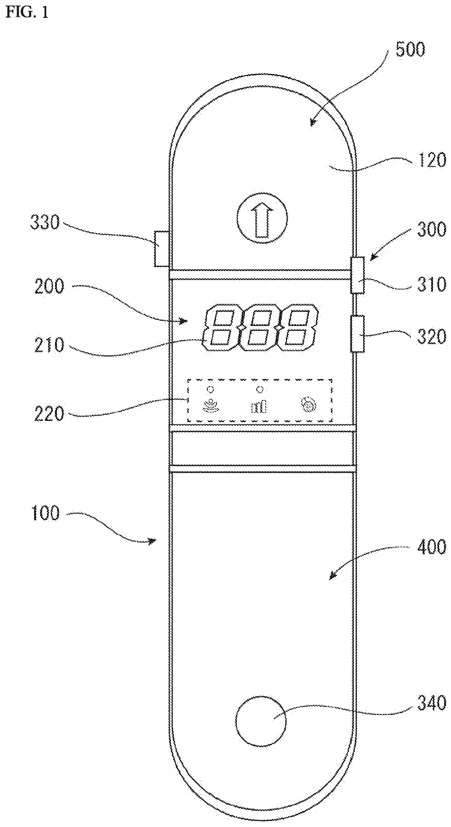

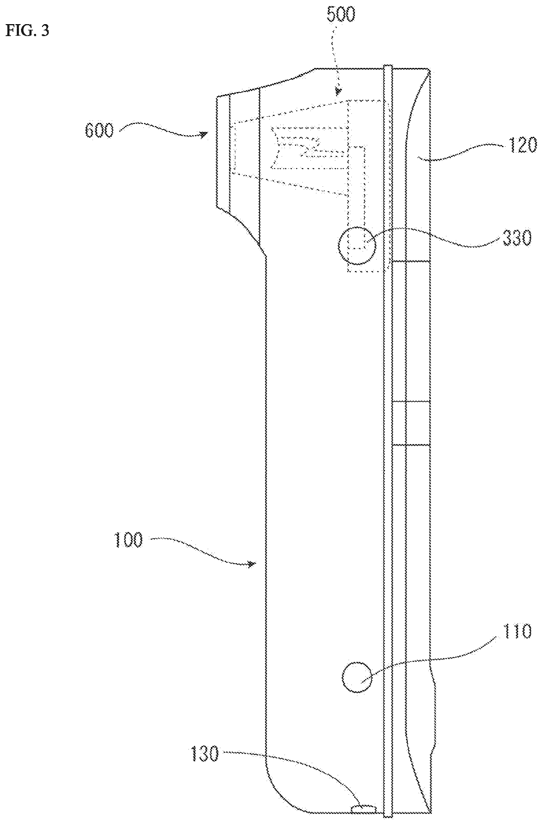

As illustrated in FIGS. 1 to 3, a main body 100 of a composite device of the present invention is provided with a first display unit 200, a button unit 300, a control-circuit unit 400, a cartridge unit 500, and a head unit 600.

As a body of a beauty instrument, the main body 100 is mounted with the cartridge unit 500 inserted into an upper portion of the main body 100 and is equipped with the first display unit 200 on a front surface. Moreover, the button unit 300 is provided respectively on left and right side surfaces of the main body 100, and inside the main body 100 is disposed the control-circuit unit 400, which is configured with a circuit for generating an ultrasound.

The first display unit 200 is equipped on the front surface of the main body 100 and is provided with an irradiation-count display unit 210, which displays a number of times the ultrasound is irradiated, and a state-display unit 220, which displays a supply state of the ultrasound or a microcurrent.

The irradiation-count display unit 210 is positioned in an upper level of the first display unit 200; when the ultrasound is irradiated to the skin, the irradiation count is counted by the control-circuit unit 400 and displayed in a numerical format on the irradiation-count display 210.

The state-display unit 220 is positioned in a lower level of the first display unit 200 and displays the supply state of the ultrasound or the microcurrent. That is, the state-display unit 220 represents in a form such as a state-display light so it can be confirmed whether a current state is an ultrasound mode or a microcurrent mode; in a situation of the ultrasound mode, it represents a state of intensity levels of a signal of an ultrasound focusing action and the ultrasound.

The button unit 300 is equipped respectively to the left and right side surfaces of the main body 100. The button unit 300 is equipped with a button 310 whereby the ultrasound mode or the microcurrent mode can be selected, a level button 320 whereby the ultrasound intensity level can be selected according to a state of the skin, and an ultrasound start button 330 whereby ultrasound irradiation matching the selected level is started.

As one example, equipping is such that when a focusing ultrasound mode is selected, the intensity of the ultrasound signal is adjusted. That is, equipping is such that the focusing ultrasound can be actuated by selecting an intensity of strong, medium, or weak. The above intensity of the focusing ultrasound can be varied by adjusting the level of the ultrasound signal or a duty ratio by current and voltage. For example, by an action of raising the level of the ultrasound signal, a high intensity can be output, and by an action of raising the duty ratio, a high intensity can be output.

Furthermore, a user can select the microcurrent mode and, when performing massaging, adhere the head unit 600 to a usage area and massage with a slow rubbing motion while using at the same time dedicated cosmetics (of, for example, a gel type). That is, by the user gripping with the hand the main body 100, which serves as a conductor, and causing a conductor unit 610 of the head unit 600 to contact the skin, a method whereby the microcurrent flows through the body of the user can be selected and used.

Note that no microcurrent can be supplied by the user to another person by the user gripping the main body 100 by hand because no circuit of the current is formed when the conductor unit 610 of the head unit 600 is caused to contact the skin of the other person. To supplement such a shortcoming, the composite device of the present invention is prepared with a separate microcurrent band and a microcurrent-band terminal 110 in a lower level of the main body 100; by having the other person wear the microcurrent band, the user can ensure that the microcurrent is supplied to the other person.

In this manner, the composite device according to the present invention can contribute to activating collagen and the like by an effect of thermal stimulation upon supplying to the dermal layer of the skin ultrasound energy for beauty. Moreover, rough skin can be massaged via the microcurrent.

Next, in the lower level of the main body 100, there is a power button 340 whereby the device can be powered on and off and a display light that indicates a state of a remaining capacity of a battery whereby a charging state of the battery can be confirmed.

By these, two objects are achieved: turning the power on and off and displaying the remaining battery capacity.

The control-circuit unit 400 is disposed inside the main body 100 and can actuate and control the ultrasound or the microcurrent.

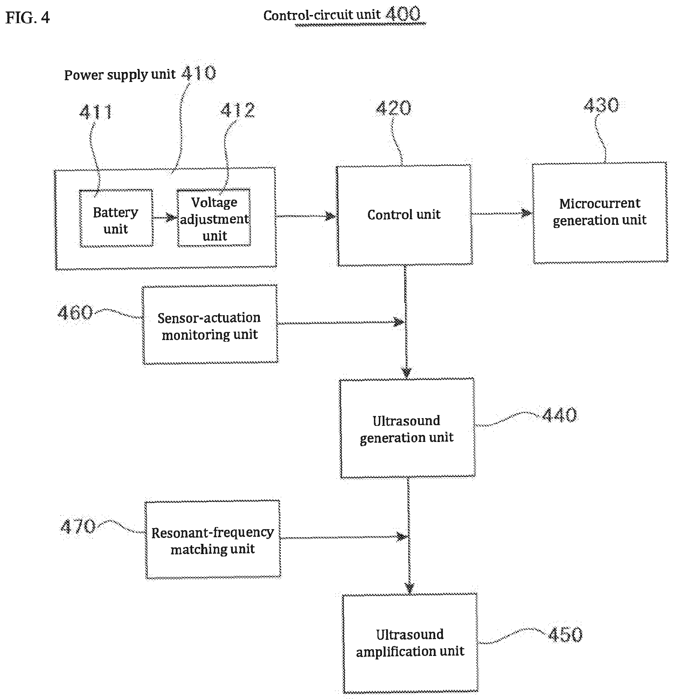

FIG. 4 is a block diagram of the control-circuit unit of the composite device according to the example of the present invention.

As illustrated in FIG. 4, the control-circuit unit 400 is provided with a power supply unit 410, a control unit 420, a microcurrent generation unit 430, an ultrasound generation unit 440, and an ultrasound amplification unit 450.

The power supply unit 410 receives power supply from an external power source via a power input terminal 130 and supplies an operating voltage to each block configuring the circuit. Moreover, the power supply unit 410 has a battery unit 411 and a voltage adjustment unit 412. The battery unit 411 stores the power supplied from the external power source and supplies this in a predetermined constant-voltage mode to the voltage adjustment unit 412. The voltage adjustment unit 412 receives the voltage of the battery unit 411 and raises or lowers this to generate a voltage necessary for operating each circuit block.

The control unit 420 receives the voltage supplied from the power supply unit 410 and controls actuation of the microcurrent generation unit 430, the ultrasound amplification unit 450, and the ultrasound generation unit 440.

For example, the control unit 420 generates a first AC signal necessary to actuate the ultrasound generation unit 440. In a situation where the ultrasound amplification unit 450 attempts to carry out a focusing and amplifying action by an ultrasound signal having a frequency of, for example, 7 MHz, the control unit 420 generates an AC signal of 7 MHz. Because of this, the control unit 420 generates a first AC signal having a first level with the frequency of 7 MHz via a starting operation by the input signal.

Furthermore, the control unit 420 controls actuation of the microcurrent by the microcurrent generation unit 430. For example, when the control unit 420 generates the first AC signal by actuating the ultrasound generation unit 440, the control unit 420 stops actuation of the microcurrent. Moreover, the control unit 420 stops actuation of the ultrasound generation unit 440 in an interval where generation of the microcurrent is actuated.

In particular, the control unit 420 can count an actuation count of the ultrasound amplification unit 450 by the ultrasound generation unit 440. That is, when the ultrasound amplification unit 450 undergoes one irradiation actuation, an ultrasound signal of a specified frequency is generated, generation of an ultrasound signal is performed by control of the control unit 420 in conjunction therewith, and generation is stopped thereafter. This is counted as one ultrasound actuation.

The microcurrent generation unit 430, depending on the control signal of the control unit 420, forms a microcurrent generation signal for supplying the microcurrent to the skin. The microcurrent generated by the microcurrent generation unit 430 is conducted upon the conductor unit 610 equipped to the head unit 600 contacting the skin. At this time, the microcurrent generated from the microcurrent generation unit 430 is set to a voltage of 2.5 V or less and a current of 40 µA or less.

By control actuation of the control unit 420, the ultrasound generation unit 440 forms an ultrasound generation signal necessary to actuate the ultrasound amplification unit 450. That is, the ultrasound generation unit 440 forms the ultrasound generation signal by the first AC signal and the control signal generated by the control unit 420. The above signal of ultrasound generation is configured by an interval having a specified frequency and an interval where only a predetermined DC level appears.

For example, in a situation where the ultrasound amplification unit 450 above performs vibration actuation by a signal of 7 MHz, the first AC signal has an aspect of a square wave of 7 MHz having the first level. Moreover, the ultrasound generation signal is set to an interval where the frequency of 7 MHz appears and the interval where the DC level appears. Therefore, one period of the ultrasound generation signal is separated into a frequency-signal interval of 7 MHz and an interval where a constant DC level appears that is a rest period.

Furthermore, the ultrasound generation unit 440 raises a level of the ultrasound generation signal above via DC-DC conversion or a voltage-raising operation and generates an ultrasound drive signal according to the raised level. The ultrasound drive signal is approved by the ultrasound amplification unit 450, and the ultrasound amplification unit 450, by the supplied ultrasound drive signal, carries out an ultrasound amplifying action that amplifies the ultrasound to a predetermined resonant frequency and supplies the amplified ultrasound signal to the skin.

The control-circuit unit 400 of the composite device of the ultrasound and the microcurrent according to the present invention can further include a sensor-actuation monitoring unit 460 that senses an actuation state when the ultrasound amplification unit 450 actuates and forms a control signal according to the actuation state and a resonant-frequency matching unit 470 that matches a deviation of a resonant frequency generated by an ultrasound-generating vibration element.

The sensor-actuation monitoring unit plays a role of sensing actuation conditions for generating the control signal of the control unit 420 by taking into consideration safety of product actuation when the ultrasound amplification unit 450 is actuated. That is, the sensor-actuation monitoring unit 460 can restrict excessive actuation of the ultrasound by monitoring, for example, a situation where a user induces abnormal usage conditions or excessive actuation on the skin.

In one example, in a situation where a state is sensed where the sensor unit equipped to the head unit 600 is not adhered to the skin, the irradiation action is stopped by sensing a condition where the amplified ultrasound is irradiated to a surface of the skin via an air layer as a medium.

Furthermore, disposed inside the main body 100 is an acceleration sensor that senses movement or a position of the main body 100. This acceleration sensor has a role of transmitting actuation displacement to the control unit 420 so the ultrasound can be irradiated evenly to the skin and sensing so the amplified ultrasound can be irradiated at a constant interval and has a function of sensing a situation in a stopped state where the amplified ultrasound signal is continuously focused and irradiated to one location on the skin and preventing such from occurring.

For example, when the ultrasound is irradiated by taking into consideration the safety of product actuation, the acceleration sensor senses the movement or the position of the main body 100; if it is sensed that the main body 100 is stopped, the ultrasound is stopped upon being irradiated once.

The resonant-frequency matching unit 470 is provided with an auto-tracing function for matching, if a deviation of the resonant frequency is found by the ultrasound amplification unit 450, the arisen deviation.

Generally, due to material characteristics thereof, ultrasound-generating vibration elements have individual resonant frequencies; because a deviation in the resonant frequency may arise in steps of manufacture and processing, respective matching work is necessary with regard to such a deviation at a time of product application. However, such work of matching resonant frequencies, while possible in a situation of producing a small amount of products, severely decreases productivity in a situation of mass production. Therefore, the composite device of the ultrasound and the microcurrent according to the present invention, by being separately equipped with the resonant-frequency matching unit 470, can match resonant frequencies by auto-tracing an optimal resonant frequency even if a deviation arises between the resonant frequency amplified by the ultrasound amplification unit 450 and the resonant frequency of the ultrasound-generating vibration element.

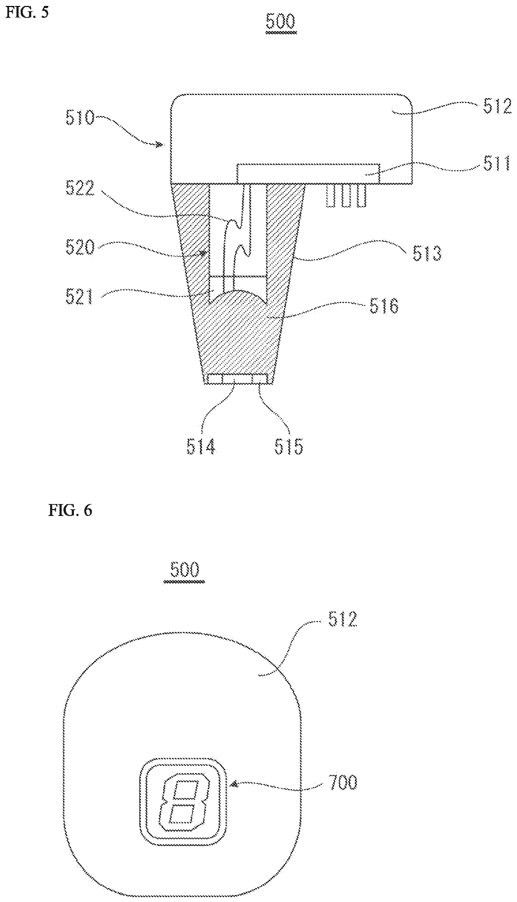

FIG. 5 is a schematic cross-sectional view of the cartridge unit according to the example of the present invention.

As illustrated in FIG. 5, the cartridge unit 500 of the composite device of the ultrasound and the microcurrent according to the present invention is mounted to the main body 100 as a detachable type. The cartridge unit 500 can be inserted into the main body 100 upon opening a cover 120 of the cartridge designed in the main-body 100 upper portion. If the cartridge unit 500 is mounted to the main body 100, a contact portion of the cartridge unit 500 is exposed to a main-body 100 back surface, and the ultrasound can be irradiated to the skin by the exposed contact portion contacting the skin.

Furthermore, as illustrated in FIG. 5, the cartridge unit 500 is provided with an external cartridge 510 that receives the ultrasound drive signal and is filled with an ultrasound medium 516 and an internal cartridge 520 that is disposed inside the external cartridge 510 and generates the ultrasound.

The external cartridge 510 is provided with a linking portion 511, a cartridge case 512, a medium reservoir 513, a transmission film 514, and a cover (ring) 515.

The linking portion 511 has one side linked to the control-circuit unit 400 of the main body 100 and another side linked to the ultrasound-generating vibration element 521 disposed in the internal cartridge 520. That is, if the linking portion 511 receives supply of an electrical signal necessary for ultrasound vibration from the main body 100, the supplied electrical signal is transmitted to the ultrasound-generating vibration element 521 via a wire 522.

In particular, the linking portion 511 is provided in a form of a printed circuit board and is fixed to the external cartridge 510. The linking portion 511 provided in the form of a printed circuit board may also be equipped with a nonvolatile memory, and information regarding a number of times the ultrasound-generating vibration element 521 of the internal cartridge 520 of the cartridge unit 500 is driven can be saved in the nonvolatile memory. The information regarding the number of times of driving saved in the nonvolatile memory is transmitted to the control unit 420, and the control unit 420 compares this with a number of times the cartridge unit 500 is driven, which is already set. If by this the number of times of driving already set is arrived at, the control unit 420 can stop actuation of a drive unit and stop actuation of the ultrasound-generating vibration element 521.

The cartridge case 512 is formed to surround the linking portion 511 and also has a function of covering so the ultrasound medium 516 of the medium reservoir 513 does not leak to the outside.

FIG. 6 is a plan view of the cartridge unit according to the example of the present invention.

As illustrated in FIG. 6, the second display unit 700, which displays a life or remaining count of the cartridge and provides information on a replacement time of the cartridge, is equipped to an upper portion of the cartridge case 512 of the cartridge unit 500 according to the present invention. That is, the second display unit 700 can display the life or the remaining count of the cartridge by utilizing the information on the drive count of the ultrasound-generating vibration element 521 saved in the nonvolatile memory of the linking portion 511 above. Therefore, during use, in a state where the cartridge unit 500 is mounted to the main body 100, the user can readily confirm the life or the remaining capacity of the cartridge displayed on the second display unit 700 by opening the cartridge cover 120 provided on the main-body 100 upper portion.

The medium reservoir 513 is mounted to a lower portion of the cartridge case 512 and is filled with the ultrasound medium 516. The medium reservoir 513 has a shape where the interior narrows the farther it is from the cartridge case 512. Generally, the device is used by the user gripping the main body 100 by hand and causing the device to contact the skin (normally the face) of the user. That is, the device is used upright for a long period; at this time, the medium inside the medium reservoir 513 filled with the ultrasound medium 516 evaporates, generating bubbles. Because the device is used upright, such bubbles are generated in a transmission-film 514 area to which the ultrasound is irradiated and reduce an effect of ultrasound irradiation.

Therefore, to compensate for this weakness due to bubble generation, the composite device according to the present invention has the medium reservoir 513 of the shape where the interior narrows the farther it is from the cartridge case 512. Desirably, by forming a lower portion of the narrowed medium reservoir 513 to be similar in size to the contact portion that contacts the skin, the problem of bubbles being generated in the transmission-film 514 area can be resolved. In fact, because the medium reservoir 513 is formed so as to widen in a cartridge-case 512 direction, a merit is had of being able to maximize an amount of the ultrasound medium 516 contained in the medium reservoir 513.

Furthermore, any ultrasound medium 516 is desirable as long as it is of a type having an impedance analogous to the skin or other tissue of the human body; in the present example, distilled water, an alcohol, or the like can be used as the ultrasound medium 516.

The transmission film 514 is disposed in a lower portion of the medium reservoir 513. The transmission film 514 carries out a function of preventing the ultrasound medium 516 from leaking to the outside and transmitting to the skin the ultrasound transmitted via the ultrasound medium 516. As a material of the transmission film 514, desirably, polyimide (PI) or polymethylpentene (TPX) can be used.

An area where the transmission film 514 is adhered is equipped with a ring-shaped cover 515 formed of a metal or a polymer material, preventing the ultrasound medium 516 from leaking to the outside, and is fixed so the transmission film 514 does not enter into the medium. Moreover, because the cover 515 is formed in the ring shape and has a cap (lid) structure relative to the transmission film 514, it can readily fix the transmission film 514 even in a narrow space in the medium reservoir 513.

The internal cartridge 520 includes the medium reservoir 513 and can include the ultrasound-generating vibration element 521.

The ultrasound-generating vibration element 521 is positioned in a lower portion of the internal cartridge 520, is of a dome shape whose center sinks to an upper-portion side, is connected to the linking portion 511 and the wire 522, and is supplied by the linking portion 511 with the electrical signal necessary for ultrasound vibration.

Furthermore, because the internal cartridge 520 including the ultrasound-generating vibration element 521 is surrounded by the ultrasound medium 516, a surface to which the wire that supplies the electrical signal of the ultrasound-generating vibration element 521 is waterproofed by being filled in with epoxy or the like to prevent contact with the ultrasound medium 516.

So as to be compatible with a portable device, the ultrasound-generating vibration element 521 can have a size where a diameter is desirably 2 mm or more and 20 mm or less. Manufacturing the size of the ultrasound-generating vibration element 521 to be smaller than 2 mm causes manufacturing problems, and manufacturing to be greater than 20 mm makes it incompatible with a portable device. Moreover, a usage frequency is desirably 4 MHz to 10 MHz and an energy is desirably 0.20 J to 1.5 J.

The ultrasound-generating vibration element 521 according to the present invention uses a method of being fixed to the lower portion of the internal cartridge 520; however, to make a position of the ultrasound-generating vibration element 521 variable up and down or left and right within the internal cartridge 520, a linear motor or a moving means of a helical (spiral) structure is equipped inside the cartridge unit 500.

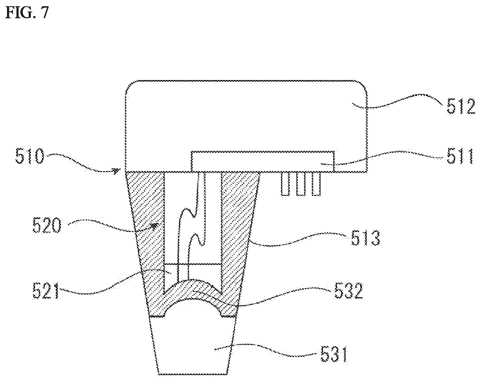

FIG. 7 is a schematic cross-sectional view of the cartridge unit according another example.

As illustrated in FIG. 7, the cartridge unit 500 according to the other example of the present invention can include a film portion 531 of a solid-phase medium in the medium reservoir 513 and an ultrasound medium 532 formed between the ultrasound-generating vibration element 521 and the film portion 531 above.

As illustrated in FIG. 7, an upper portion of the film portion 531 has a dome shape where a center portion is convex so as to correspond to the shape of the ultrasound-generating vibration element 521 and is provided with an interval of a predetermined distance from the ultrasound-generating vibration element 521 and a lower portion has a flat shape so as to be able to contact the skin and is made to be of a similar size to an inner diameter of the lower portion of the medium reservoir 513 so the ultrasound medium 532 does not leak to the outside.

The film portion 531 is a solid-phase medium, desirably a silicone material.

Furthermore, the ultrasound medium 532 formed between the ultrasound-generating vibration element 521 and the film portion 531 above is configured by a medium of a liquid component such as a silicone oil or DI (deionized) water.

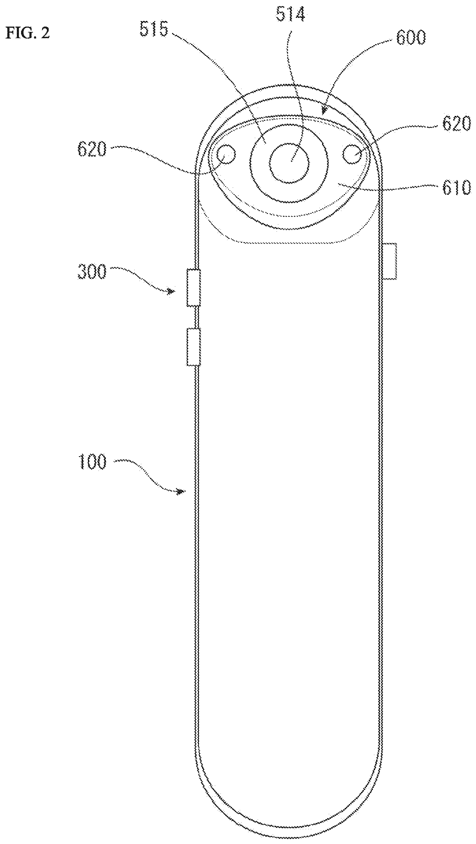

Next, referring to FIG. 2, the head unit 600 is positioned in an upper level of the main-body back surface of the main body 100, protruding in a horizontal direction on a back-surface side, and supplies to the skin the ultrasound or the microcurrent generated via the control-circuit unit 400.

The head unit 600 has the conductor unit 610 for flowing the microcurrent and a sensor unit 620 for sensing contact with the skin. Moreover, the transmission film 514 of the cartridge unit 500, which is mounted inserted through the upper portion of the main body 100, and the cover 515 are exposed to a central portion of the head unit 600 and can contact the skin together with the conductor unit 610 and the sensor unit 620 above. Therefore, the user can irradiate the ultrasound to the skin by contacting the transmission film 514 and the cover 515 positioned in the central portion of the head unit 600 to the skin and supply the microcurrent to the skin by contacting the conductor unit 610 with the skin.

Furthermore, the sensor unit 620 may be disposed on both sides of the transmission film 514 so as to be able to sense contact with the skin and the sensor unit 620 is configured from an optical sensor or a contact sensor. That is, the ultrasound is irradiated only when contact with the skin is sensed by either of the sensor units 620 equipped on the two sides.

As described above, a composite beauty instrument as a portable instrument is provided in the present invention. By this, the user can beautify the skin without restriction on location and, besides beautifying the skin via ultrasound focusing, perform massaging utilizing the microcurrent.

Note that the examples of the present invention disclosed in the present specification and the drawings merely illustrated specific examples to aid in the understanding of the present invention and are not meant to limit the scope of the present invention. Based on normal knowledge in the technical field of the present invention, it is obvious that besides the examples disclosed herein, other modified examples based on the technical idea of the present invention can also be worked.

### REFERENCE SIGNS LIST

- 100: Main body
- 110: Microcurrent-band terminal
- 120: Cartridge cover
- 130: Power input terminal
- 200: First display unit
- 210: Count display unit
- 220: State display unit
- 300: Button unit
- 310: Button
- 320: Level button
- 330: Start button
- 400: Control-circuit unit
- 410: Power supply unit
- 411: Battery unit
- 412: Voltage adjustment unit
- 420: Control unit
- 430: Microcurrent generation unit
- 440: Ultrasound generation unit
- 450: Ultrasound amplification unit
- 460: Sensor-actuation monitoring unit
- 470: Resonant-frequency matching unit
- 500: Cartridge unit
- 510: External cartridge
- 511: Linking portion
- 512: Cartridge case
- 513: Medium reservoir
- 514: Transmission film
- 515: Ring (cover)
- 516: Ultrasound medium
- 520: Internal cartridge
- 521: Ultrasound-generating vibration element
- 522: Wire
- 531: Film portion
- 532: Ultrasound medium
- 600: Head unit
- 610: Conductor unit
- 620: Sensor unit
- 700: Second display unit

## Claims

1. A composite device of an ultrasound and a microcurrent for skin care, comprising:
a main body;
a first display unit equipped to a main-body front surface;
a button unit equipped to a main-body side surface;
a control-circuit unit disposed inside the main body;
a cartridge unit mounted inserted into a main-body upper portion; and
ahead unit that is equipped to the main body and is for irradiating an ultrasound or a microcurrent to the skin.

2. The composite device of an ultrasound and a microcurrent for skin care according to claim 1, wherein the head unit is provided with a conductor unit for flowing the microcurrent by contacting the skin and a sensor unit that senses contact with the skin, wherein the sensor unit may consist of an optical sensor or a contact sensor and a configuration may be such that the ultrasound is irradiated only in a situation where contact with the skin is sensed by the sensor unit.

3. The composite device of an ultrasound and a microcurrent for skin care according to claim 1, wherein the cartridge unit is configured from an external cartridge filled with an ultrasound medium and an internal cartridge that is disposed inside the external cartridge, receives an ultrasound drive signal, and generates the ultrasound,
wherein any medium among a silicone gel, a silicone oil, and deionized water may be filled between an ultrasound-generating vibration element of the external cartridge and a film portion.

4. The composite device of an ultrasound and a microcurrent for skin care according to claim 3, wherein the external cartridge has a linking portion that receives from the main body supply of an electrical signal necessary for ultrasound vibration, a medium reservoir filled with the ultrasound medium, and a cartridge case that has the linking portion provided thereto and is mounted to the medium reservoir so the ultrasound medium does not leak to the outside, the composite device further comprising:
a transmission film that transmits to the skin the ultrasound, which is transmitted through the ultrasound medium; and
a ring that prevents the ultrasound medium from leaking to the outside and fixes the transmission film,
wherein the medium reservoir may be of a shape where an interior narrows the farther it is from the cartridge case.

5. The composite device of an ultrasound and a microcurrent for skin care according to claim 4, wherein the internal cartridge is provided with an ultrasound-generating vibration element of a dome shape where an upper-portion side is expanded in an arc, wherein the ultrasound-generating vibration element may be provided with a size where a diameter is 2 mm or more and 20 mm or less.

6. The composite device of an ultrasound and a microcurrent for skin care according to claim 1, wherein the first display unit is provided with an irradiation-count display unit that displays a number of times the ultrasound is irradiated and a state-display unit that displays a supply state of the ultrasound or the microcurrent.

7. The composite device of an ultrasound and a microcurrent for skin care according to claim 4, further comprising:
a second display unit in a cartridge-case upper portion that displays a number of remaining ultrasound irradiations of the cartridge and provides information on a cartridge replacement time.

8. The composite device of an ultrasound and a microcurrent for skin care according to claim 1, wherein the cartridge unit is detachable from the main body.

9. The composite device of an ultrasound and a microcurrent for skin care according to claim 1, wherein disposed inside the main body is an acceleration sensor that senses movement or a position of the main body and the acceleration sensor senses the movement or the position of the main body when the ultrasound is irradiated, the ultrasound being stopped upon being irradiated once in a situation where it is sensed that the movement of the main body is stopped.

10. The composite device of an ultrasound and a microcurrent for skin care according to claim 1, wherein the control-circuit unit is provided with
a power supply unit that receives power supply from an external power source and supplies an operating voltage to each block configuring a circuit;
a control unit that receives the voltage supplied from the power supply unit and controls actuation of the ultrasound or the microcurrent;
a microcurrent generation unit that forms a microcurrent generation signal for supplying the microcurrent to the skin; and
an ultrasound generation unit that forms an ultrasound generation signal to supply the ultrasound to the skin and an ultrasound amplification unit that receives the signal of the ultrasound generation unit and amplifies the ultrasound to match a predetermined resonant frequency.

11. The composite device of an ultrasound and a microcurrent for skin care according to claim 10, wherein the control-circuit unit is provided with a sensor-actuation monitoring unit that senses an actuation state of the ultrasound amplification unit and transmits a control signal according to the actuation state.

12. The composite device of an ultrasound and a microcurrent for skin care according to claim 10, wherein the control-circuit unit is provided with an auto-tracing function for matching, in a situation where a deviation in the resonant frequency arises in the ultrasound amplification unit, the arisen deviation.

13. The composite device of an ultrasound and a microcurrent for skin care according to claim 10, wherein the power supply unit has a battery unit that saves the power supplied from the external power source and a function of receiving supply of a voltage from the battery unit and raising or lowering the supplied voltage.

14. The composite device of an ultrasound and a microcurrent for skin care according to claim 10, wherein the control unit has a function of
stopping actuation of the microcurrent generation unit if the ultrasound generation unit actuates and
stopping actuation of the ultrasound generation unit if the microcurrent generation unit actuates.

15. The composite device of an ultrasound and a microcurrent for skin care according to claim 10, wherein the control unit has a function of counting an actuation count of the ultrasound amplification unit that actuates according to the ultrasound generation signal.
